# EUROPEAN PATENT SPECIFICATION

(11) **EP 4 279 414 B1**
(45) Date of publication and mention of the grant of the patent: **22.01.2025**
(21) Application number: 22871062.0
(22) Date of filing: 18.11.2022
(51) Int. Cl.: B65D 75/32, B65D 75/52, G01N 33/48

(54) **MULTI-ROW TESTING DEVICE**
MEHRREIHIGE PRÜFVORRICHTUNG
DISPOSITIF DE TEST À RANGÉES MULTIPLES

(30) Priority: 08.04.2022 CN 202210370196; 26.04.2022 CN 202210447619
(43) Date of publication of application: 22.11.2023
(73) Proprietor: Acon Biotech (Hangzhou) Co., Ltd., Hangzhou, Zhejiang 310030 (CN)
(72) Inventor: CUI, Chao, Hangzhou, Zhejiang 310030 (CN); LING, Yun, Hangzhou, Zhejiang 310030 (CN); LI, Tianhao, Hangzhou, Zhejiang 310030 (CN); WANG, Chunming, Hangzhou, Zhejiang 310030 (CN)
(74) Representative: Gulde & Partner
(86) International application number: PCT/CN2022/132931
(87) International publication number: WO 2023/193446

(56) References cited:
- CN-A- 101 031 480
- CN-A- 101 257 870
- CN-A- 102 132 158
- CN-A- 102 149 613
- CN-A- 102 803 091
- CN-A- 114 563 557
- CN-U- 204 789 585
- CN-U- 205 854 944
- CN-U- 205 854 944
- CN-U- 207 060 848
- CN-U- 211 732 447
- CN-U- 211 845 668
- CN-Y- 2 329 621
- FR-A- 1 354 112
- JP-A- 2013 220 841
- JP-A- 2014 118 193
- US-A- 2 700 556
- US-A1- 2007 144 922
- US-A1- 2008 197 045
- US-A1- 2012 080 353
- US-B1- 6 520 329
- CHANTIPMANEE NATTAPONG; SONSA-ARD THITAPORN; FUKANA NUTNAREE; KOTAKANOK KAMOLCHANOK; MANTIM THITIRAT; WILAIRAT PRAPIN; HAUSER PETE: "Contactless conductivity detector from printed circuit board for paper-based analytical systems", TALANTA, vol. 206, 5 August 2019 (2019-08-05), NL , XP085810612, ISSN: 0039-9140, DOI: 10.1016/j.talanta.2019.120227
- GUTERMAN SIDNEY; WEN XIN; GUDAVALLI GANESH; RHAJBHANDARI PRAVAKAR; DHAKAL TARA P.; WILT DAVID; KLOTZKIN DAVID: "Optimized flexible cover films for improved conversion efficiency in thin film flexible solar cells", OPTICAL MATERIALS, vol. 79, 12 April 2018 (2018-04-12), NL , pages 243 - 246, XP085394818, ISSN: 0925-3467, DOI: 10.1016/j.optmat.2018.03.034
- WANG YIN-BIAO: "Research Advance of Immunochromatographic Strip Test", JOURNAL OF HENAN UNIVERSITY OF SCIENCE & TECHNOLOGY (MEDICAL SCIENCE), vol. 35, no. 3, 30 September 2017 (2017-09-30), pages 236 - 240, XP093097922
- HONG LIANG: "Analysis of the Blister Packaging Technology", PACKAGING ENGINEERING, vol. 29, no. 2, 29 February 2008 (2008-02-29), pages 73 - 75, XP093097926

## Description

### Field of the Invention

The present invention relates to a detection device, in particular to a multi-lined detection device suitable for automated production.

### Background of the Invention

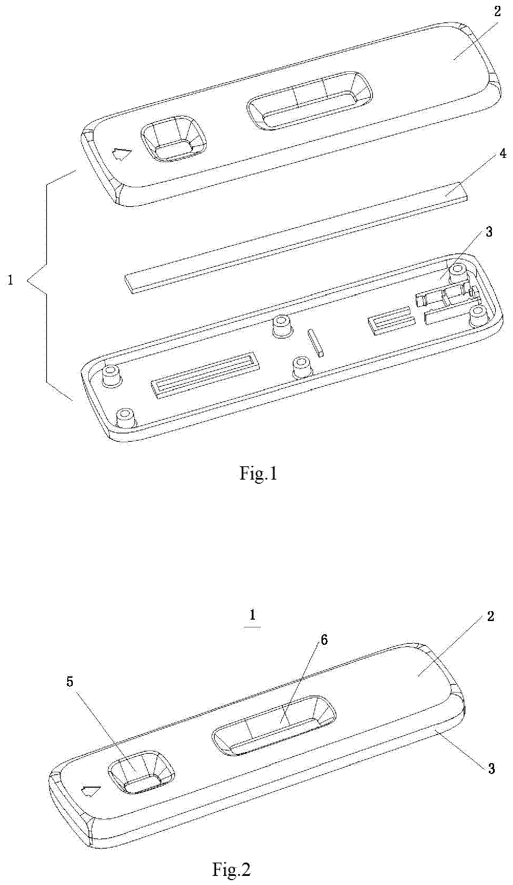

Disposable detection kits for detection of diseases or other physical conditions using body fluids such as urine, blood or other tissue fluids of a human body have been commonly used all over the world, and their applications can be in laboratories operated by professionals, or at home, schools, shopping malls, road checkpoints, customs, etc. operated by non-professionals themselves. As shown in Fig. 1 and Fig. 2, such a detection kit 1 generally includes an upper cover 2, a bottom plate 3 and a test strip 4, wherein the upper cover 2 and the bottom plate 3 are combined in such a manner as buckling, welding, glue adhering or the like, and the test strip 4 is arranged on the bottom plate 3 and located between the upper cover 2 and the bottom plate 3. The upper cover 2 includes a sample addition hole 5 and an observation hole 6. A liquid sample for detection is applied to a test strip via the sample addition hole 5, and after the end of the detection, a detection result on the test strip is observed via the observation hole 6, and determined accordingly.

The upper cover 2 and the bottom plate 3 of such detection kit as shown in Fig. 1 and Fig. 2, are made of plastic articles that are thermally injection molded, and they account for a large portion of the cost of the whole detection kit. Both the upper cover 2 and the bottom plate 3 have a certain height, and after their combination, the size of the whole detection kit 1 increases, and is much larger than the size of the test strip 4, increasing storage and transportation costs. Moreover, due to long degradation time of plastics, usage of a large amount of plastics does not comply with the concept of environmental protection. Such detection kit 1 has been packaged in an aluminum foil bag in factory to avoid bumping against the test strips in the detection kit 1 during transportation; and an operator has to tear the aluminum foil bag when the kit is to be used, and then takes out of the detection kit 1 for detection, so that the operation steps are tedious. Furthermore, such detection detection kit is not suitable for manufacturing by automated equipment, but requires a large amount of human power for manual assembly, the assembly process is boring and cumbersome, and long time of operation is prone to resulting in fatigue, burnout and errors, reducing the qualification rate of products, and increasing labor costs.

As the disposable detection kits have been widely used, in order to reduce the purchase costs of users, it is necessary to meet these needs with the lowest possible product cost and convenient usage mode. Particularly in the case of spreading of epidemic diseases, how to quickly promote such detection kits so that more people can afford to use and are willing to use such rapid detection kit products is a problem that needs to be solved at present.

For example, CN 205 854 944 U discloses a test paper class bubble cap formula packaging structure including: at least one aluminium foil bubble cap unit. CN 211 732 447 U relates to a packaging plate, in particular to a novel capsule medicine aluminum-plastic packaging plate including a substrate, wherein the base plate is rectangular; a plurality of rectangular openings which are formed in the base plate and every two adjacent rectangular openings are separated through a partition plate. Each rectangular opening is internally provided with a medicine bin used for containing capsule medicine. JP 2013220841 A describes a blister package which has a plurality of packaging units comprising a storage section for storing an item and a flange surrounding the storage section formed by attaching top and bottom two sheets. Each of the adjoining packaging units is mutually joined through a weakened line to be separated by each flange between the units. A boundary whose length is at least a half of that of the weakened line and which is wide enough to be separable from the storage section is formed, and the two sheets of the package are not adhered in the boundary. US 2012/080353 A1 discloses a retail and storage pack of the blister card type for zinc-air button cells including a support, and a transparent plastics film fixed thereon and having cup-shaped stamp-outs for reception of the button cells, wherein the pack is divided into single pack regions which can respectively be torn off separately and each of which respectively comprises at least one of the cup-shaped stamp-outs, and wherein the support is provided with an adhesive layer to which button cells received in the cup-shaped stamp-outs adhere by their base, which bases are provided with air inlet openings.

An objective of the present invention is to provide a multi-lined detection device, by which automated production is easy to realize. The device is small in size, is low in production, packaging and transportation costs, and uses little plastic materials, so that it is very environmentally friendly. When the multi-lined detection device is split into at least two single detection devices, the sealed packaging of adjacent detection devices is not affected.

### Summary of the Invention

In order to solve the above technical problems, the present invention provides a multi-lined detection device, comprising a bottom card, at least two test strips located above the bottom card and arranged side by side, and at least two sealing films located above the bottom card and the test strips, the thickness of the bottom card is greater than the thickness of the sealing film. Wherein, the at least two sealing films each form a cavity with the bottom card in the area where the test strip is located, and a bonding band connecting said sealing film to the bottom card is formed around the cavity. The test strip is received in the cavity. The bottom card forms a cutting line, which is incompletely disconnected, in an area between two adjacent bonding bands. The two adjacent sealing films are completely disconnected in the area between the two adjacent bonding bands.

Further improvement of the present invention lies in that the incompletely disconnected cutting line on the bottom card comprises a discontinuous cutting line, a continuous cutting line or a combination thereof; the discontinuous cutting line is formed by connecting segments spaced in sequence and fractures penetrating through the bottom card; and the continuous cutting line is a cutting line not penetrating through the bottom card.

Further improvement of the present invention lies in that the length of the connecting segment of the discontinuous cutting line ranges from 0.1mm to 3.0mm, the length of the fracture of the discontinuous cutting line ranges from 0.5mm to 10.0mm, and the cutting depth of the continuous cutting line ranges from one fifth to four fifths of the thickness of the bottom card.

Further improvement of the present invention lies in that the bottom card has a first end and a second end in a direction parallel to the cutting line, wherein the shortest distance from the first end to the bonding band is greater than the shortest distance from the second end to the bonding band, and the bottom card forms a completely disconnected opening, which is "U" shaped or "V" shaped, at the junction of the first end thereof and the cutting line.

Further improvement of the present invention lies in that the bonding band bonding the sealing film to the bottom card is closed, and the cavity for receiving the test strip is sealed by the closed bonding band.

Further improvement of the present invention lies in that the closed bonding band comprises an intermediate portion and two ends connected to the two ends of the intermediate portion, respectively, wherein the width of at least the end close to the first end of the bottom card gradually decreases in the direction from the intermediate portion of the bonding band to the two ends of the bonding band. Further improvement of the present invention lies in that the intermediate portion is two straight lines parallel to each other, and at least the end close to the first end of the bottom card is of a circular structure or a triangular structure without a bottom edge or a trapezoidal structure without a bottom edge.

Further improvement of the present invention lies in that a completely disconnected notch is formed between two adjacent sealing films.

Further improvement of the present invention lies in that the test strip is bonded to the bottom card through viscose.

Further improvement of the present invention lies in that the bottom card is made of a PE or PP material with the thickness ranging from 0.1mm to 0.5mm; and the sealing film is made of a PE or PP or PET material with the thickness ranging from 0.05mm to 0.20mm.

### Beneficial effects

The present is small in size and uses a little amount of materials, so that the packaging and transportation costs are low. Furthermore, since a relatively flexible material that can be rolled is used, it is suitable for automated production, greatly reducing labor intensity and production costs. In addition, since the two adjacent sealing films are completely disconnected, when the bottom card is torn, the adjacent sealing films are not torn and the sealed packaging of the adjacent detection devices is not affected.

Moreover, in the technical solution of the present invention that the sealing film is completely cut off, when two adjacent detection devices are torn, the tearing force cannot be transferred to the sealing films of the adjacent detection devices because the two adjacent sealing films are completely disconnected and have no connection between each other and generate no implication between them, thus not affecting the bonding formed by the adjacent sealing films and the bottom card, so that the sealed packaging of the adjacent detection devices will not be damaged.

Further, the present invention forms a completely disconnected notch between the two adjacent sealing films, and the completely disconnected notch provides convenience for detection of whether the two adjacent sealing films are completely disconnected in the area between the two adjacent bonding bands, as the formation of the completely disconnected notch is more convenient for visual observation. This design improves the probability of complete disconnection between two adjacent sealing films and reduces the probability of affecting adjacent sealing films when a single detection device is torn, resulting in better beneficial effects.

### Brief Description of the Drawings

Fig. 1 is a stereoscopic exploded view of a detection kit in the prior art;
Fig. 2 is a stereoscopic schematic view of Fig. 1 after combination;
Fig. 3 is an exploded view of a first embodiment of a multi-lined detection device of the present invention;
Fig. 4 is a partially combined view of Fig. 3;
Fig. 5 is a top view of the multi-lined detection device of the present invention after removal of sealing films;
Fig. 6 is a top view of the sealing film of the multi-lined detection device of the present invention;
Fig. 7 is a top view of Fig. 3 after combination (the parts obscured by the sealing films are shown by dashed lines);
Fig. 8 is a partially enlarged schematic view within circle Ain Fig. 5;
Fig. 9 is a partially enlarged schematic view within circle B in Fig. 6;
Fig. 10 is a partially enlarged schematic view within circle Cin Fig. 7;
Fig. 11 is a sectional view of Fig. 7 along line A-A;
Fig. 12 is a partially enlarged view within circle Din Fig. 11;
Fig. 13 is schematic view of a modified design of Fig. 12;
Fig. 14 is a schematic view of another modified design of Fig. 12;
Fig. 15 is a schematic view of another modified design of Fig. 12;
Fig. 16 is schematic view of a modified design of Fig. 5;
Fig. 17 is a partially enlarged schematic view of a part within circle E in Fig. 16 along line B-B;
Fig. 18 is a schematic view of a modified design of Fig. 8;
Fig. 19 is a schematic view of another modified design of Fig. 8;
Fig. 20 is a top view of a single detection device of after removal of sealing films, in which the shadow lines represent a bonding band;
Fig. 21 is schematic view of a modified design of Fig. 20;
Fig. 22 is a schematic view of another modified design of Fig. 20;
Fig. 23 is a schematic view of another modified design of Fig. 20;
Fig. 24 is a partially enlarged schematic view within circle F in Fig. 20;
Fig. 25 is a partially enlarged schematic view within circle G in Fig. 21;
Fig. 26 is a partially enlarged schematic view within circle H in Fig. 22;
Fig. 27 is a partially enlarged schematic view within circle I in Fig. 23;
Fig. 28 is a schematic view of a modified design of Fig. 3; and
Fig. 29 is a schematic top view after one of the detection devices in the multi-lined detection device of the present invention is torn off.

### Detailed Description of the Embodiments

The technical solutions of the present invention will be further specified below through embodiments in conjunction with the drawings.

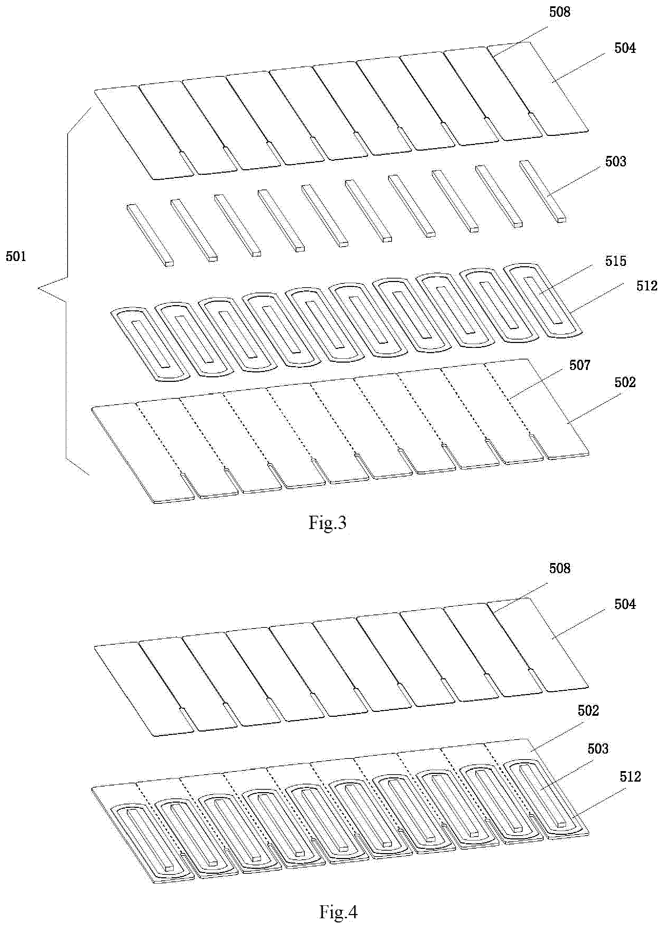

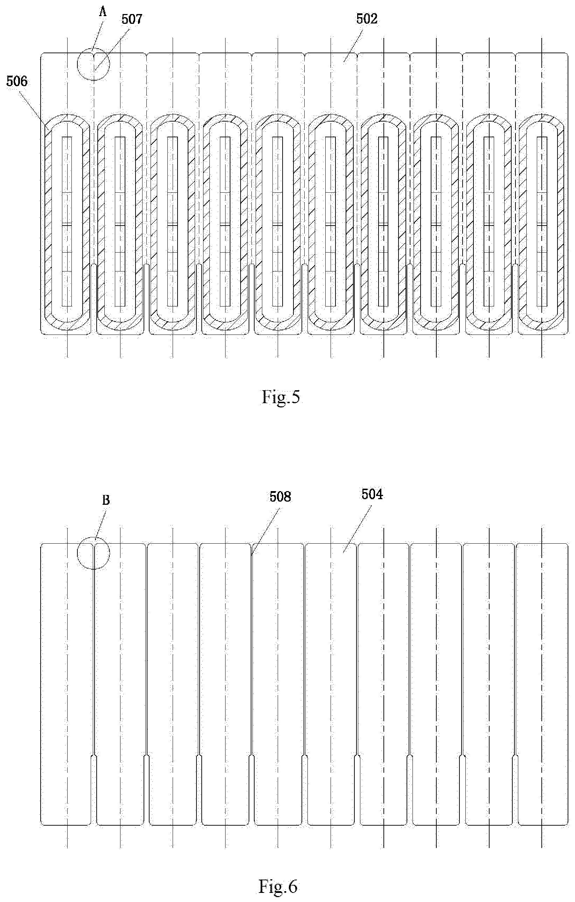

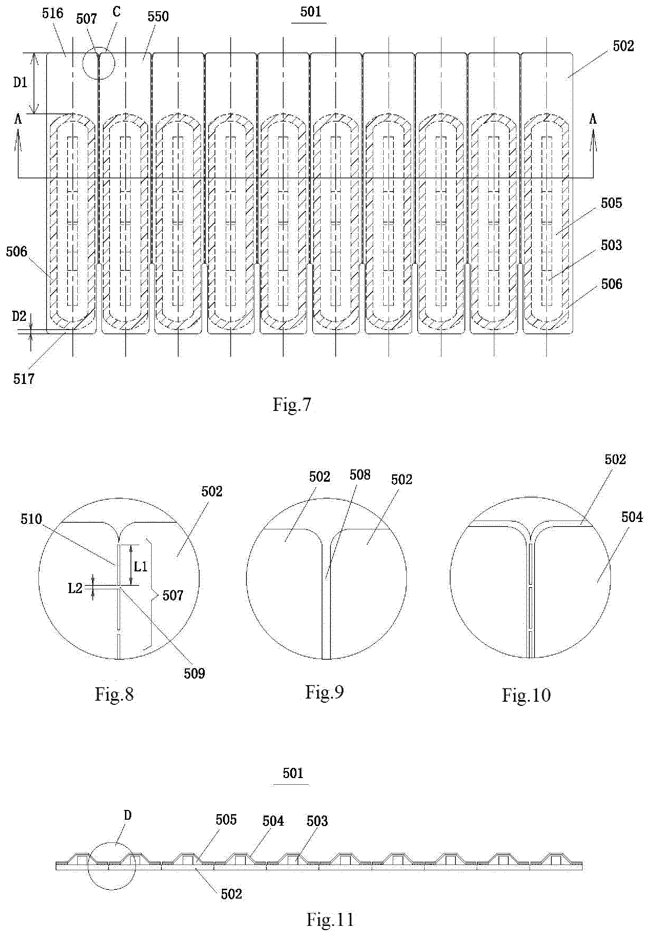

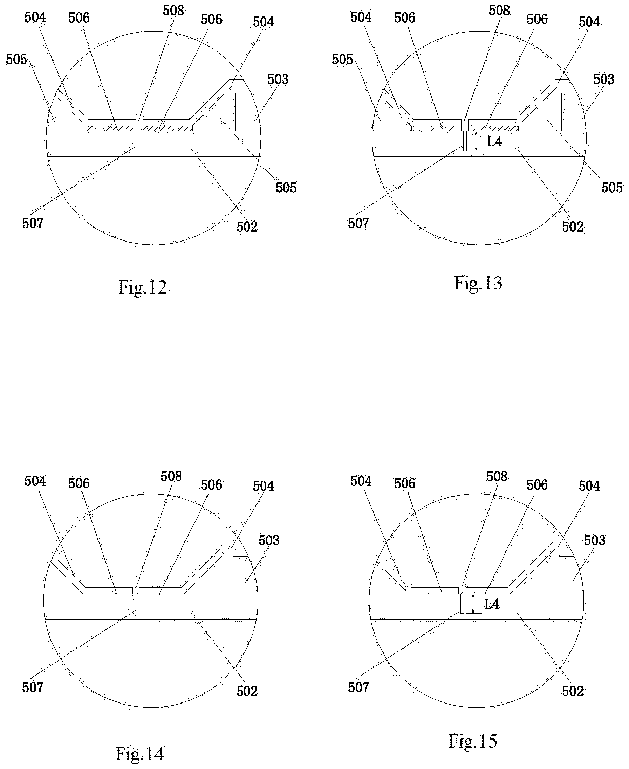

Please refer to Figs. 3 to 29, the present invention relates to a multi-lined detection device 501, including a bottom card 502, at least two test strips 503 located above the bottom card and arranged side by side, and at least two sealing films 504 located above the bottom card and the test strips. Wherein, these sealing films 504 each form a cavity 505 with the bottom card 502 in the area where the test strip 503 is located, and a bonding band 506 connecting said sealing film to the bottom card is formed around the cavity (please refer to Figs. 12 to 15). The test strip 503 is received in the cavity 505. The bottom card 502 forms an incompletely disconnected cutting line 507 (as shown by the dashed line in Fig. 5) in an area between two adjacent bonding bands 506. In the embodiments shown in Figs. 12 to 15, two adjacent sealing films 504 are completely disconnected in the area between the two adjacent bonding bands 506 to form a completely disconnected notch 508. The notch 508 causes the two sealing films 504 to form a gap in the area between the two adjacent bonding bands 506, as shown in Fig. 9. The gap may be between 0.05mm to 2.0mm, preferably, 0.1mm to 0.5mm. The design has the advantages that when two adjacent detection devices are torn, the tearing force cannot be transferred to the sealing films 504 of the adjacent detection devices because the two adjacent sealing films 504 are completely disconnected and have no connection between each other and generate no implication between them, thus not affecting the bonding formed by the adjacent sealing films 504 and the bottom card 502, so that the sealed packaging of the adjacent detection devices will not be damaged. Further, the completely disconnected notch 508 provides convenience for detection of whether the two adjacent sealing films 504 are completely disconnected in the area between the two adjacent bonding bands 506, as the formation of the completely disconnected notch 508 is more convenient for detection through visual observation (not only including detection through human visual observation but also including detection through automated visual observation of equipment). This design improves the reliability of completely disconnecting two adjacent sealing films 504 and reduces the probability of affecting adjacent sealing films 504 when a single detection device is torn, resulting in better beneficial effects. The test strip 503 is bonded to the bottom card 502 through viscose 515.

The bottom card 502 is preferably made of a moisture-proof and oxidation-resistant PE or PP material, with its thickness preferably ranging from 0.1mm to 0.5mm, more preferably ranging from 0.2mm to 0.35mm. The sealing film 504 is preferably made of a moisture-proof and oxidation-resistant PE or PP or PET material, with its thickness preferably ranging from 0.05mm to 0.20mm, more preferably ranging from 0.08mm to 0.15mm. Since the thickness of the bottom card 502 is much greater than the thickness of the sealing film 504, the bottom card 502 has moderate rigidity and higher strength, while the sealing film 504 is softer and has better stretching resistance and tearing resistance performances, and both of them are suitable for producing products of the present invention on automated equipment.

In the embodiment shown in Fig. 8, the incompletely disconnected cutting line 507 on the bottom card 502 is a discontinuous cutting line, which is formed by connecting segments 509 spaced in sequence and fractures 510 penetrating through the bottom card. The longer the connecting segment 509 is, the better the connecting stability is (the lower the probability of improper separation of the bottom card 502 is), but the larger the difficulty of separating the bottom card 502 along the incompletely disconnected cutting line 507 is; and in contrary, the shorter the connecting segment 509 is, the worse the connecting stability is (the higher the probability of improper separation of the bottom card 502 is), but the smaller the difficulty of separating the bottom card 502 along the incompletely disconnected cutting line 507 is. Therefore, it is necessary to properly select the lengths of the connecting segment 509 and the fracture 510. Preferably, the length L1 of the fracture 510 of the discontinuous cutting line ranges from 0.5mm to 10.0mm (ranging from value A to value B means greater than or equal to value A and less than or equal to value B, the same in the full text), and the length L2 of the connecting segment 509 of the discontinuous cutting line ranges from 0.1mm to 3.0mm; and more preferably, the length L1 of the fracture 510 of the discontinuous cutting line ranges from 1.0mm to 5.0mm, and the length L2 of the connecting segment 509 of the discontinuous cutting line ranges from 0.5mm to 1.5mm.

The side of the bottom card 502 facing the sealing film 504 is an upper surface, the back side of which is a lower surface. In the embodiments shown in Figs. 13, 15 and 17, the incompletely disconnected cutting line 507 on the bottom card 502 is a continuous cutting line not penetrating through the upper and lower surfaces of the bottom card. Preferably, the cutting depth L4 of this continuous cutting line ranges from one fifth to four fifths of the thickness L5 of the bottom card. The continuous cutting line is preferably located on the upper surface of the bottom card, but may also be located on the lower surface of the bottom card.

In another embodiment (not shown in the figure), the incompletely disconnected cutting line 507 on the bottom card 502 may also be a combination of the discontinuous cutting line and the continuous cutting line as previously described, i.e., one part of the incompletely disconnected cutting line 507 is the discontinuous cutting line as described in Figs. 8, 12 and 14 and the other part is the continuous cutting line as shown in Figs. 13, 15 and 17.

In the embodiments shown in Figs. 12 to 15, the at least two adjacent sealing films 504 are bonded to the bottom card 502 at the edge of its area between the two adjacent bonding bands 506. This embodiment has the advantages that the multi-lined detection device 501 in the present invention is compact in structure and small in size.

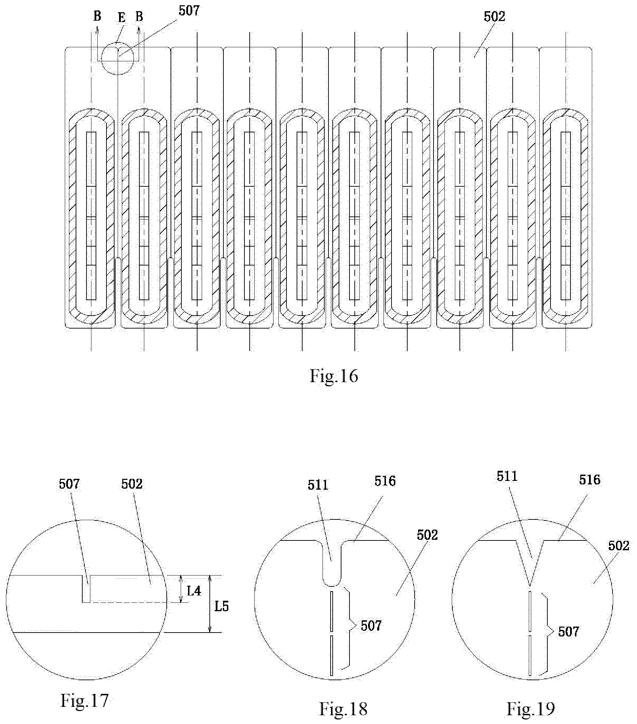

Please refer to Fig. 7, the bottom card 502 has a first end 516 and a second end 517 in a direction parallel to the incompletely disconnected cutting line 507, wherein the shortest distance "D1" from the first end 516 to the bonding band 506 is greater than the shortest distance "D2" from the second end 517 to the bonding band 506. As shown in Figs. 18 and 19, the bottom card 502 forms a completely disconnected opening 511, which is "U" shaped (as shown in Fig. 18) or "V" shaped (as shown in Fig. 19), at the junction of the first end 516 thereof and the incompletely disconnected cutting line 507. The purpose of providing the completely disconnected opening 511 is to use the completely disconnected opening 511 as a starting point for tearing, thereby facilitating separation of the two adjacent single detection devices 550. The first end 516 typically serves as a holding end for an operator to tear off the sealing film 504 from the bottom card 502. Preferably, the completely disconnected opening 511 is located at the first end 516 of the bottom card 502, thus after tearing off the detection device from the completely disconnected opening 511, the sealing film 504 can be torn from the bottom card 502 directly without changing hands to expose the test strip 503 for detection, instead of changing hands and subsequently turning over the torn single detection device 550 (inverted) to tear the sealing film 504 from the bottom card 502, so that the operation is more convenient.

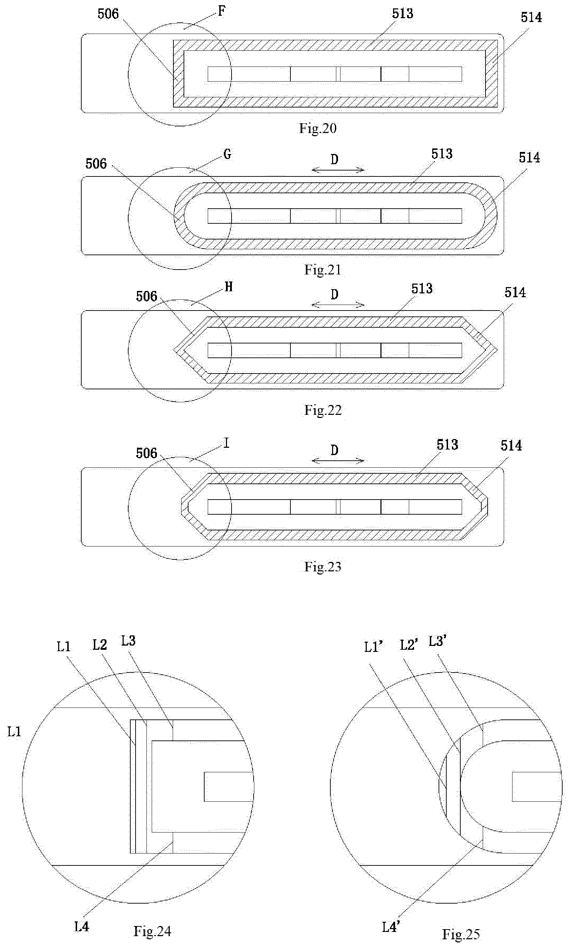

Please refer to Figs. 7, 11 and Figs. 20 to 23, the bonding band 506 that bonds the sealing film 504 to the bottom card 502 is a closed ring, and the cavity 505 receiving the test strip 503 is sealed by the closed bonding band 506. The closed ring described in the present invention means that the bonding band 506 is closed, rather than limiting its shape to a circular ring; and on the contrary, the structure may be as shown in Figs. 20 to 23 or may be a structure developed according to Figs. 20 to 23. In one embodiment, the bonding band 506 is formed by a binder 512 (e.g., glue or double-sided adhesive tape, etc.) (as shown in Fig. 3). In another embodiment, no binder is used and the bonding band 506 is formed by fusing the bottom card 502 and the sealing film 504 under pressure and heat (as shown in Figs. 14, 15 and 28). In another embodiment, the bottom card 502 has a thermally adhesive film on its side facing the sealing film 504, the thermally adhesive film bonding the sealing film 504 to the bottom card 502 when being subjected to pressure and heat, so as to form said bonding band 506 (not shown in the figure).

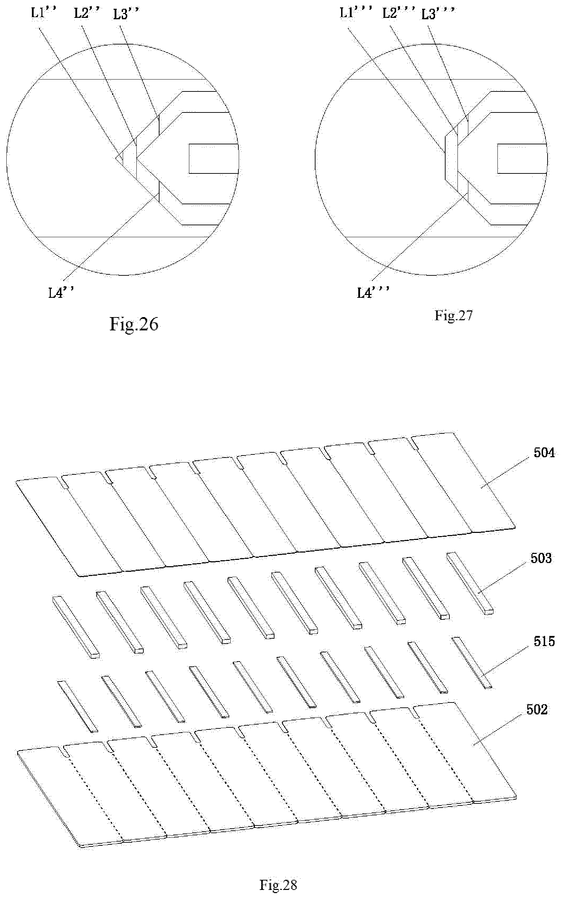

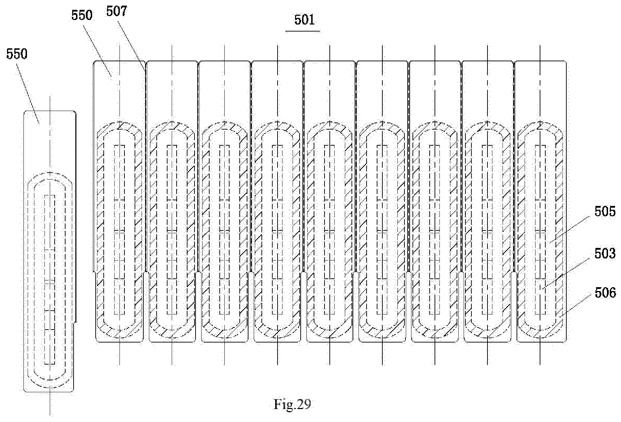

Please refer to Figs. 20 to 27, the closed bonding band 506 includes an intermediate portion 513 and two ends 514 connected to the two ends of the intermediate portion, respectively. The intermediate portion 513 preferably is two straight lines parallel to each other. In the embodiments shown in Fig. 20 and Fig. 24, the width of the end 514 is substantially constant. In the embodiment shown in Figs. 21 to 23 and Figs. 25 to 27, the width of the end 514 gradually decreases from the intermediate portion of the bonding band to the two ends of the bonding band (in the direction shown by arrow "D" in Figs. 21 to 23). Preferably, the end 514 is of a circular structure (as shown in Figs. 21 and 25) or a triangular structure without a bottom edge (as shown in Figs. 22 and 26) or a trapezoidal structure without a bottom edge (as shown in Figs. 23 and 27). In another embodiment, the two ends 514 may also be a combination of any of the structures as shown in Figs. 21 to 23. In contrast to the embodiment shown in Fig. 24, the embodiments shown in Figs. 25 to 27 have the advantages that the lengths of tear lines L1', L2', (L3'+L4'), tear lines L1", L2‴, (L3"+L4") and tear lines L1‴, L2'", (L3" + L4") are smaller than the lengths of tear lines L1, L2, and (L3+L4) in Fig. 24, respectively, i.e., L1' < L1, L2' < L2, (L3' + L4') < (L3+L4), and so on for others. This has the advantages that in the tearing process, the bonding force between the sealing film 504 and the bottom card 502 is smaller, so that it is easier to tear and separate the sealing film 504 from the bottom card 502. Fig. 29 is a schematic view of tearing off a single detection device 550 from the multi-lined detection device 501 along the incompletely disconnected cutting line 507.

The above description is only the specific implementations of the present invention, and is not thereby limiting the scope claimed for protection by the present invention.

## Claims

1. A multi-lined detection device (501), comprising a bottom card (502), at least two test strips (503) located above the bottom card (502) and arranged side by side, and at least two sealing films (504) located above the bottom card (502) and the test strips (503), the thickness of the bottom card (502) is greater than the thickness of the sealing film (504), wherein the at least two sealing films (504) each form a cavity (505) with the bottom card (502) in the area where the test strip (503) is located, and a bonding band (506) connecting said sealing film (504) to the bottom card (502) is formed around the cavity (505); the test strip (503) is received in the cavity (505); and the bottom card (502) forms a cutting line, which is incompletely disconnected, in an area between two adjacent bonding bands (506); and two adjacent sealing films (504) are completely disconnected in the area between the two adjacent bonding bands (506).

2. The multi-lined detection device (501) of claim 1, wherein the incompletely disconnected cutting line (507) on the bottom card (502) comprises a discontinuous cutting line, a continuous cutting line or a combination thereof; the discontinuous cutting line is formed by connecting segments (509) spaced in sequence and fractures (510) penetrating through the bottom card (502); and the continuous cutting line is a cutting line not penetrating through the bottom card (502).

3. The multi-lined detection device (501) of claim 2, wherein the length of the connecting segment (509) of the discontinuous cutting line ranges from 0.1mm to 3.0mm, the length of the fracture (510) of the discontinuous cutting line ranges from 0.5mm to 10.0mm, and the cutting depth of the continuous cutting line ranges from one fifth to four fifths of the thickness of the bottom card (502).

4. The multi-lined detection device (501) of claim 2, wherein the bottom card (502) has a first end (516) and a second end (517) in a direction parallel to the cutting line, wherein the shortest distance from the first end (516) to the bonding band (506) is greater than the shortest distance from the second end (517) to the bonding band (506), and the bottom card (502) forms a completely disconnected opening (511), which is "U" shaped or "V" shaped, at the junction of the first end (516) thereof and the cutting line.

5. The multi-lined detection device (501) of claim 4, wherein the bonding band (506) bonding the sealing film (504) to the bottom card (502) is closed, and the cavity (505) for receiving the test strip (503) is sealed by the closed bonding band (506).

6. The multi-lined detection device (501) of claim 5, wherein the closed bonding band (506) comprises an intermediate portion (513) and two ends (514) connected to the two ends (514) of the intermediate portion (513), respectively, wherein the width of at least the end (514) close to the first end (516) of the bottom card (502) gradually decreases in the direction from the intermediate portion (513) of the bonding band (506) to the two ends (514) of the bonding band (506).

7. The multi-lined detection device (501) of claim 6, wherein the intermediate portion (513) is two straight lines parallel to each other, and at least the end (514) close to the first end (516) of the bottom card (502) is of a circular structure or a triangular structure without a bottom edge or a trapezoidal structure without a bottom edge.

8. The multi-lined detection device (501) of claim 1, wherein a completely disconnected notch (508) is formed between two adjacent sealing films (504).

9. The multi-lined detection device (501) of claim 1, wherein the test strip (503) is bonded to the bottom card (502) through viscose (515).

10. The multi-lined detection device (501) of claim 1, wherein the bottom card (502) is made of a PE or PP material with the thickness ranging from 0.1mm to 0.5mm; and the sealing film (504) is made of a PE or PP or PET material with the thickness ranging from 0.05mm to 0.20mm.

## Patentansprüche

1. Mehrreihige Nachweisvorrichtung (501), umfassend eine untere Karte (502), mindestens zwei Teststreifen (503), die über der unteren Karte (502) liegen und nebeneinander angeordnet sind, und mindestens zwei Abdichtfilme (504), die über der unteren Karte (502) und den Teststreifen (503) liegen, wobei die Dicke der unteren Karte (502) größer ist als die Dicke des Abdichtfilms (504), wobei die mindestens zwei Abdichtfilme (504) jeweils einen Hohlraum (505) mit der unteren Karte (502) in dem Bereich ausbilden, in dem sich der Teststreifen (503) befindet, und ein Verklebungsband (506), das den Abdichtfilm (504) mit der unteren Karte (502) verbindet, um den Hohlraum (505) herum ausgebildet ist; der Teststreifen (503) in dem Hohlraum (505) aufgenommen ist; und die untere Karte (502) eine Trennlinie, die unvollständig abgetrennt ist, in einem Bereich zwischen zwei benachbarten Verklebungsbändern (506) bildet; und zwei benachbarte Abdichtfilme (504) in dem Bereich zwischen den beiden benachbarten Verklebungsbändern (506) vollständig abgetrennt sind.

2. Mehrreihige Nachweisvorrichtung (501) nach Anspruch 1, wobei die unvollständig abgetrennte Trennlinie (507) an der unteren Karte (502) eine unterbrochene Trennlinie, eine durchgängige Trennlinie oder eine Kombination daraus umfasst; die unterbrochene Trennlinie von räumlich beabstandeten Verbindungssegmenten (509) und Unterbrechungen (510), die durch die untere Karte (502) hindurchgehen, ausgebildet wird, und die durchgängige Trennlinie eine Trennlinie ist, die nicht durch die untere Karte (502) hindurchgeht.

3. Mehrreihige Nachweisvorrichtung (501) nach Anspruch 2, wobei die Länge des Verbindungssegments (509) der unterbrochenen Trennlinie von 0,1 mm bis 3,0 mm reicht, die Länge der Unterbrechung (510) der unterbrochenen Trennlinie von 0,5 mm bis 10,0 mm reicht, und die Trenntiefe der durchgängigen Trennlinie von einem Fünftel bis vier Fünftel der Dicke der unteren Karte (502) reicht.

4. Mehrreihige Nachweisvorrichtung (501) nach Anspruch 2, wobei die untere Karte (502) ein erstes Ende (516) und ein zweites Ende (517) in einer Richtung parallel zur Trennlinie aufweist, wobei die kürzeste Entfernung vom ersten Ende (516) zum Verklebungsband (506) größer ist als die kürzeste Entfernung vom zweiten Ende (517) zum Verklebungsband (506), und die untere Karte (502) eine vollständig abgetrennte Öffnung (511), die "U"-förmig oder "V"-förmig ist, an der Verbindung des ersten Endes (516) davon und der Trennlinie ausbildet.

5. Mehrreihige Nachweisvorrichtung (501) nach Anspruch 4, wobei das Verklebungsband (506), das den Abdichtfilm (504) mit der unteren Karte (502) verklebt, geschlossen ist, und der Hohlraum (505) zum Aufnehmen des Teststreifens (503) mit dem geschlossenen Verklebungsband (506) abgedichtet ist.

6. Mehrreihige Nachweisvorrichtung (501) nach Anspruch 5, wobei das geschlossene Verklebungsband (506) einen Zwischenabschnitt (513) und zwei Enden (514) umfasst, die jeweils mit den zwei Enden (514) des Zwischenabschnitts (513) verbunden sind, wobei die Breite von zumindest dem Ende (514) nahe am ersten Ende (516) der unteren Karte (502) in der Richtung vom Zwischenabschnitt (513) des Verklebungsbands (506) zu den zwei Enden (514) des Verklebungsbands (506) allmählich schmaler wird.

7. Mehrreihige Nachweisvorrichtung (501) nach Anspruch 6, wobei der Zwischenabschnitt (513) zwei gerade Linien parallel zueinander sind und zumindest das Ende (514) nahe am ersten Ende (516) der unteren Karte (502) kreisförmig oder dreieckig ohne untere Kante oder trapezförmig ohne untere Kante ist.

8. Mehrreihige Nachweisvorrichtung (501) nach Anspruch 1, wobei eine vollständig abgetrennte Kerbe (508) zwischen zwei benachbarten Abdichtfilmen (504) ausgebildet ist.

9. Mehrreihige Nachweisvorrichtung (501) nach Anspruch 1, wobei der Teststreifen (503) mittels Viskose (515) mit der unteren Karte (502) verklebt ist.

10. Mehrreihige Nachweisvorrichtung (501) nach Anspruch 1, wobei die untere Karte (502) aus einem PE- oder PP-Material mit der Dicke von 0,1 mm bis 0,5 mm gefertigt ist und der Abdichtfilm (504) aus einem PE- oder PP- oder PET-Material mit der Dicke von 0,05 mm bis 0,20 mm gefertigt ist.

## Revendications

1. Dispositif de détection à lignes multiples (501), comprenant une carte inférieure (502), au moins deux bandelettes réactives (503) situées au-dessus de la carte inférieure (502) et agencées côte à côte, et au moins deux films d'étanchéité (504) situés au-dessus de la carte inférieure (502) et des bandelettes réactives (503), l'épaisseur de la carte inférieure (502) est supérieure à l'épaisseur du film d'étanchéité (504), dans lequel les au moins deux films d'étanchéité (504) forment chacun une cavité (505) avec la carte inférieure (502) dans la zone où la bandelette réactive (503) est située, et une bande de liaison (506) reliant ledit film d'étanchéité (504) à la carte inférieure (502) est formée autour de la cavité (505) ; la bandelette réactive (503) est reçue dans la cavité (505) ; et la carte inférieure (502) forme une ligne de coupe, qui est déconnectée de manière incomplète, dans une zone entre deux bandes de liaison adjacentes (506) ; et deux films d'étanchéité adjacents (504) sont déconnectés entièrement dans la zone entre les deux bandes de liaison adjacentes (506).

2. Dispositif de détection à lignes multiples (501) selon la revendication 1, dans lequel la ligne de coupe déconnectée de manière incomplète (507) sur la carte inférieure (502) comprend une ligne de coupe discontinue, une ligne de coupe continue ou une combinaison de celles-ci ; la ligne de coupe discontinue est formée en reliant des segments (509) espacés en séquence et en fractures (510) pénétrant à travers la carte inférieure (502) ; et la ligne de coupe continue est une ligne de coupe qui ne pénètre pas à travers la carte inférieure (502).

3. Dispositif de détection à lignes multiples (501) selon la revendication 2, dans lequel la longueur du segment de liaison (509) de la ligne de coupe discontinue est comprise de 0,1 mm à 3,0 mm, la longueur de la fracture (510) de la ligne de coupe discontinue est comprise de 0,5 mm à 10,0 mm, et la profondeur de coupe de la ligne de coupe continue est comprise de un cinquième à quatre cinquièmes de l'épaisseur de la carte inférieure (502).

4. Dispositif de détection à lignes multiples (501) selon la revendication 2, dans lequel la carte inférieure (502) présente une première extrémité (516) et une seconde extrémité (517) dans une direction parallèle à la ligne de coupe, dans lequel la distance la plus courte à partir de la première extrémité (516) jusqu'à la bande de liaison (506) est supérieure à la distance la plus courte à partir de la seconde extrémité (517) jusqu'à la bande de liaison (506), et la carte inférieure (502) forme une ouverture entièrement déconnectée (511), qui a une forme en « U » ou une forme en « V », à la jonction de la première extrémité (516) de celle-ci et de la ligne de coupe.

5. Dispositif de détection à lignes multiples (501) selon la revendication 4, dans lequel la bande de liaison (506) reliant le film d'étanchéité (504) à la carte inférieure (502) est fermée, et la cavité (505) destinée à recevoir la bandelette réactive (503) est scellée par la bande de liaison fermée (506).

6. Dispositif de détection à lignes multiples (501) selon la revendication 5, dans lequel la bande de liaison fermée (506) comprend une portion intermédiaire (513) et deux extrémités (514) reliées aux deux extrémités (514) de la portion intermédiaire (513), respectivement, dans lequel la largeur au moins de l'extrémité (514) à proximité de la première extrémité (516) de la carte inférieure (502) diminue petit à petit dans la direction à partir de la portion intermédiaire (513) de la bande de liaison (506) jusqu'aux deux extrémités (514) de la bande de liaison (506).

7. Dispositif de détection à lignes multiples (501) selon la revendication 6, dans lequel la portion intermédiaire (513) est formée de deux lignes droites parallèles l'une à l'autre, et au moins l'extrémité (514) à proximité de la première extrémité (516) de la carte inférieure (502) présente une structure circulaire ou une structure triangulaire sans bord inférieur ou une structure trapézoïdale sans bord inférieur.

8. Dispositif de détection à lignes multiples (501) selon la revendication 1, dans lequel une encoche entièrement déconnectée (508) est formée entre deux films d'étanchéité adjacents (504).

9. Dispositif de détection à lignes multiples (501) selon la revendication 1, dans lequel la bandelette réactive (503) est reliée à la carte inférieure (502) par le biais de viscose (515).

10. Dispositif de détection à lignes multiples (501) selon la revendication 1, dans lequel la carte inférieure (502) est réalisée en un matériau PE ou PP avec une épaisseur comprise dans la plage de 0,1 mm à 0,5 mm ; et le film d'étanchéité (504) est réalisé en un matériau PE ou PP ou PET avec une épaisseur comprise dans la plage de 0,05 mm à 0,20 mm.
